Europäisches Patentamt

European Patent Office

Office européen des brevets

(11) Publication number: **0 334 679**
**A2**

# (12) EUROPEAN PATENT APPLICATION

(21) Application number: 89303001.5

(22) Date of filing: 28.03.89

(51) Int. Cl.⁴: **A 61 L 2/08**
A 01 N 1/02, A 61 K 35/16

(30) Priority: 25.03.88 GB 8807187

(43) Date of publication of application:
27.09.89 Bulletin 89/39

(84) Designated Contracting States:
CH DE FR GB IT LI NL SE

(71) Applicant: NATIONAL RESEARCH DEVELOPMENT
CORPORATION
101 Newington Causeway
London SE1 6BU (GB)

(72) Inventor: Harrison, Jean Florence
The North East Thames Regional Transfusion Centre
Crescent Drive Brentwood Essex CM15 8DP (GB)

Mann, George Forbes
Appartment 708 118 Monroe Street
Rockville Maryland (US)

Zuckerman, Arie Jeremy
The London School of Hygiene and Tropical Medicine
Keppel Street London WC1E 7HT (GB)

(74) Representative: Hamilton, Raymond
Patent Department National Research Development
Corporation 101 Newington Causeway
London SE1 6BU (GB)

(54) Improvements in or relating to the inactivation of infectious agents.

(57) Blood plasma or plasma derived products or other body tissues especially those suitable for transplantation can be irradiated at temperatures below the ambient in order to inactivate infectious agents. Irradiation at temperatures of -20° to -60°C using dosages in the range 20 to 60 KGy is preferred. The procedure inactivates infectious agents whilst reducing damage to proteinaceous material.

EP 0 334 679 A2

Bundesdruckerei Berlin

## Description

### IMPROVEMENTS IN OR RELATING TO THE INACTIVATION OF INFECTIOUS AGENTS

This invention relates to the treatment of blood products and of other forms of body tissue, particularly for the purpose of inactivating infectious agents which may be present therein.

Blood transfusion and the use of blood products is an essential part of modern medicine but it has been recognised for a long time that a number of infectious agents can be transmitted in this way. Even though systematic screening is routinely applied to blood donations to detect a variety of infectious agents or antibodies thereto, some risk of transmission must necessarily remain due to limitations on test sensitivity and specificity, and the possibility of donations having been collected at an early stage in the incubation period of a disease. This problem has been increasingly highlighted in the past few years with the rapid emergence of the Acquired Immune Deficiency Syndrome (AIDS) and the identification of the human Immunodeficiency virus (HIV) as the causative agent.

The blood obtained from donors is frequently fractionated into plasma and a number of cellular and acellular products. The plasma is either administered to patients as such or in the form of cryoprecipitate or is processed further to yield valuable proteinaceous materials such as the clotting factors, for example Factor VIII (antihemophilic factor, AHF) and Factor IX (Christmas factor), and albumin. Few suitable methods are available for the inactivation of infectious agents in blood products. Thus, most of the commonly used procedures, such as treatment with aldehydes, have secondary effects on enzymes and labile proteins and also result in chemical contamination of the products. Consequently, emphasis has been placed on physical methods such as heat inactivation. However, although the inactivation of viruses by dry or wet heat is currently applied to purified products in the dry, usually lyophilized, state such as Factor VIII and other blood clotting factors, the procedure is difficult because of the inherent thermolabililty of the components. Wet protein-containing products cannot be treated with dry or wet heat without denaturation of the proteins. Accordingly, despite the importance of avoiding infection through the administration of plasma or products derived therefrom. plasma is not currently treated in order to inactivate infectious agents and, instead, tests are applied to check on the absence of such agents. Thus, commercial screening tests for the detection of antibodies to HIV are now available from a number of manufacturers but there is considerable variation between these tests in terms of specificity, sensitivity and reliability. Further, the incubation period for HIV is known to be long and variable, and may include periods without detectable antibody markers of infection. On this basis, it is clear that screening alone cannot prevent completely the transmission of the virus through blood and blood products.

In other areas than the treatment of blood products irradiation has been used as an alternative sterilizing agent to heat. A combination of a chemical agent, beta-propiolactone, and ultraviolet radiation has been successfully used to inactivate hepatitis B virus in plasma, as reported by Prince et al, Reviews of Infectious Diseases, 1983, 5 92-107, but Spire et al, Lancet, 1985 i, 188-189 reports HIV to be only slightly sensitive to ultraviolet radiation so that high doses are required to achieve the necessary level of inactivation. Gamma irradiation has generally been regarded as being of little value in the area of blood products apart from the prevention of graft versus host disease (GVH) in immunosuppressed or immunocompromised patients. Thus Button et al, TRANSFUSION, 1981 21, 419-426 report the effects of gamma radiation on blood components in the context of the avoidance of GVH disease but the maximum dosages used were 0.2 KGy (20,000 rads) whilst the recommended dosages were less than this, such doses being very low compared to those required for effective viral inactivation. Spire et al, Lancet, 1985, i, 188-189, report the effect of gamma irradiation on lymphocytes in tissue culture infected with HIV and note that a dosage of 2 KGy ($2 \times 10^5$ rads) did not produce inactivation. Although inactivation was observed at higher levels, this work did not involve studies of any concomitant effect of the irradiation on proteins. Horowitz et al, TRANSFUSION, 1985, 25, 516-522 report the effect of gamma irradiation on a lyophilised Factor VIII concentrate and demonstrate the inactivation of vesicular stomatitis and Sindbis viruses at a dose of 10 KGy (1.0 megarads). However, at higher doses the detrimental effect of the irradiation on the proteins was more marked and the amount of Factor VIII retained in the irradiated sample was found to decrease to unacceptable levels.

It has now been found that it is possible to utilise radiation to inactivate infectious agents such as HIV without having an unacceptable detrimental effect on other radiation sensitive materials such as proteins, provided the irradiation is carried out at a temperature which is less than ambient temperature.

Accordingly the present invention comprises a method for the treatment of blood plasma or a plasma- derived product or other body tissues, which comprises subjecting the plasma, tissue or derived product to gamma radiation at a temperature which is less than ambient temperature.

It is found, quite surprisingly, that by lowering the temperature of irradiation from ambient temperature it is possible, whilst not appreciably altering the inactivating effect of the radiation on viruses, significantly to reduce its detrimental effect on proteins, thereby allowing the administration of quite high dosage levels without destruction of proteins such as the clotting factors and albumin present in plasma.

The present invention is applicable to a variety of body tissues, its particular value lying in the treatment of tissues which are transferable from one host to another. However, even at a temperature less than ambient temperature a degree of radiation damage may be sustained by certain cells, for example some white blood cells, and for this reason the invention is of particular interest for application to plasma or other body tissue which is not constituted of viable cellular material, or to plasma derived products. Such tissue of a non-viable form, i.e. not constituted of cells which are capable of division and growth, may, for example by ossified or calcified. The method of the invention may also be used to inactivate infectious agents such as HIV in tissues constituted of viable cellular material, either alone or when contaminating non-viable cellular material, but the viable cellular material may suffer an impairment, albeit possibly only a temporary one, of its function.

As indicated previously, the invention is of particular interest in the treatment of blood plasma. Blood plasma is obtained from anticoagulated whole blood by the removal of the cellular elements of the blood leaving the residual fluid which constitutes the plasma. The blood plasma which is treated by the method of the present invention may be obtained from whole blood by any suitable procedure, particularly conventional procedures which are based upon sedimentation. In the United Kingdom methods utilising centrifugation to enhance sedimentation are employed to separate whole blood into the four fractions of plasma, platelet concentrate, white cell (leucocyte) concentrate and leucocyte-poor packed red cells using a closed bag system.

The present invention is not however limited to the treatment of raw plasma which is the direct product of the treatment of the whole blood but can also be applied to plasma-derived products. in particular, the method of the invention may be applied to the product known as cryoprecipitate which is obtained by thawing frozen plasma at just above 0°C and separating the residue, which is rich in Factor VIII, from the supernatant. It should be appreciated, however, that cryoprecipitate is still a liquid rather than a solid product and the value of the invention, as regards the treatment of plasma derived products, does lie in the treatment of products which are liquid at ambient temperature, for example at 25°C. Although in principle it is possible to apply the method to plasma-derived products, for example those containing one or more of the clotting factors, which are further refined than cryoprecipitate and which are therefore often solid at ambient temperature, it is far more convenient to treat the whole of the plasma, or at least the cryoprecipitate or some other relatively impurified product which is liquid at ambient temperature, at the outset rather than treating separately different refined products obtained from the plasma. The real value of the invention lies in the possibility, which was not hitherto available, of treating plasma or a plasma-derived product which is in a liquid state, being relatively unpurified, for example having been subjected to no more than centrifugation and separation, optionally followed by a freeze/thaw treatment. Such treatment can be used to effect virus inactivation at the outset rather than treating separately individual products isolated from the plasma.

It will be appreciated that the present invention can be used to treat both human and animal tissues, plasma from various animals, for example cattle, being of value as a source of proteins. Apart from the treatment of blood products, the invention is of especial interest in relation to the treatment of human body tissues for transplantation. Once again, the invention is of particular value for the inactivation of HIV which might be present in blood or tissue fluid contaminating the surface of the tissue to be transplanted. Examples include particularly non-viable cellular tissue, for example teeth and bone, especially the ear ossicles, and also tendons and especially corneas, so that in distinction to the situation with blood products, the preferred materials for treatment will be of a solid form.

Accordingly, although the following description is directed particularly to the treatment of plasma it will be appreciated that it applies equally to the treatment of plasma-derived products and of other body tissues, particularly transplantable tissues such as those just described.

The irradiation is conveniently effected at a temperature of 0°C or less and for liquid materials such as plasma the temperature is preferably such that the material is frozen. The temperature required to maintain plasma in a frozen state is about -10°C, although it is usually subjected to a temperature of about -60°C to effect freezing and is stored at about -40°C. The protection of proteins in any material against the effect of irradiation is enhanced by decreasing the temperature below -10°C and a preferred maximum temperature for the irradiation of plasma or any other material is -20° or -30°C, conveniently −40°C. Whilst it is believed that the temperature can be reduced further quite significantly whilst still achieving virus deactivation, for example to -180°C or even more, the use of temperatures as low as this is expensive in terms of the cooling required and in practice a temperature no lower than -80°C is preferred, conveniently no lower than -60° or -50°C. Accordingly a preferred range of temperature for effecting irradiation of the plasma or other material is -20° to -60°C, particularly -30° to −50°C, for example about -40°C. It will be appreciated that the irradiation may produce some temperature rise in the irradiated material and continued cooling of the material during irradiation will usually be appropriate. A convenient procedure is for the plasma or plasma-derived product such as cryoprecipitate to be frozen in the usual way and then to be subjected to irradiation before storage in the frozen state (transplantable tissue may be taken from storage in the frozen state for treatment or fresh tissue may be frozen for direct irradiation).

Since one of the reasons for requiring a treatment for the inactivation of potential viral contamination in plasma and plasma-derived products is the fact that virus screening procedures are not totally reliable, it is preferred to use a dosage of gamma radiation which is capable of inactivating any level of the relevant viral contamination which is likely to be encountered. Moreover, although infectious agents such as those causing hepatitis B and non A non B hepatitis and cytomegalovirus (CMV), human T-cell lymphotrophic virus-I (HTLVI) and parvovirus, may cause problems in the blood product area, there is no doubt that the current major problem is posed by HIV. With the aim of HIV inactivation in view, therefore, a convenient minimum dose of radiation is 20 or 25 KGy (kilograys). It is believed that little advantage accrues from the use of a dosage in excess of 60 or 50 KGy although a minimum dosage of 40 KGy is preferred since our findings suggest that a dose of 40 KGy should be sufficient to inactivate an amount of $10^{6.5}$ ID of HIV/ml plasma (ID = infectious Doses). It has been estimated that the maximum contamination of a unit of plasma (i.e. of the plasma derived from one whole blood donation) could be $10^6$ ID of HIV/ml, although the likelihood of such a level of contamination arising is not great. Accordingly, therefore, a preferred dosage range is 20 to 60 KGy, conveniently 25 or 30 to 50, for example 40 KGy or more.

We have found that, whilst 40 KGy will inactivate high levels of HIV contamination, the corresponding loss of clotting factors produced by this dose of radiation is acceptable, the percentage losses of activity observed at this irradiation level being 14.6% for Factor VIII:C (procoagulant), 38.8% for Factor VIII:VWF (von Willebrand Factor) and 23.3% for Factor IX. Experiments carried out with a range of other viruses which are not normal plasma contaminants suggest that the method of the present invention should not necessarily only be capable of inactivating HIV in plasma and it is quite possible that it may also be effective in inactivating infectious agents such as that causing non A non B hepatitis.

Any suitable source of gamma radiation may be used, convenient gamma emitters being $Co^{60}$ and $U^{235}$. The dosage rate may be selected over a wide range, a slow rate causing less heating effect but requiring a longer period of use of the gamma ray source. A range of 1 or 2 to 20 or 40 KGFy/hour is often suitable, conveniently of 4 to 15 KGy/hour. A convenient procedure involves an automated system which presents the frozen plasma or plasma-derived product to the irradiation for a suitable period. Once the plasma or plasma-derived product has been irradiated it may be stored for subsequent use or used immediately, either for administration to patients or for processing to produce various purified products.

The use of a temperature above 0°C, or indeed above -10°C, is of more interest with body tissues other than plasma, for example the various transplantable tissues discussed hereinbefore. It is even possible with such tissues to contemplate irradiation at ambient temperature, such a procedure providing a further aspect of the present invention.

The invention is illustrated by the following Example.

## Example: Effect of gamma irradiation on virus activity and on coagulation factor activity

Fresh plasma was separated within 6 hours from whole blood obtained in single blood donations and was then pooled under aseptic conditions. The pooled plasma was divided into aliquots of approxiamtely 280 ml in volume in unused sterile single plasma packs. The packs were either virus seeded with HIV, strain RF, obtained from H9 cells which was inoculated into the plasma to give a level of contamination of approximately $10^6$ ID HIV/ml, or were left uninfected, both types being blast frozen at -60°C and then stored at -40°C prior to irradiation.

Both uninfected and infected plasma samples were gamma irradiated on a bed of cardice in the frozen state at about -40°C using $U^{235}$ spent reactor fuel rods as the gamma source. The doses administered, predetermined by ionisation chamber, were 0 (control), 2.5, 5, 10, 20 and 40 KGy, the dosage rate being 12 KGy per hour. Uninfected plasma samples were also gamma irradiated on a bed of cardice in the frozen state at about -40°C in two separate series of experiments using a cobalt 60 source. The target doses corresponded to part or all of the range of doses just described but the doses achieved, as measured using perspex dosimeters, were 0,-,-, 15.9, 28.9 and 44 in the first series and 0, 3.1, 6.3, 13, 22 and 42 KGy in he second series, the dosage rate being 5.5 KGy/hour in each case.

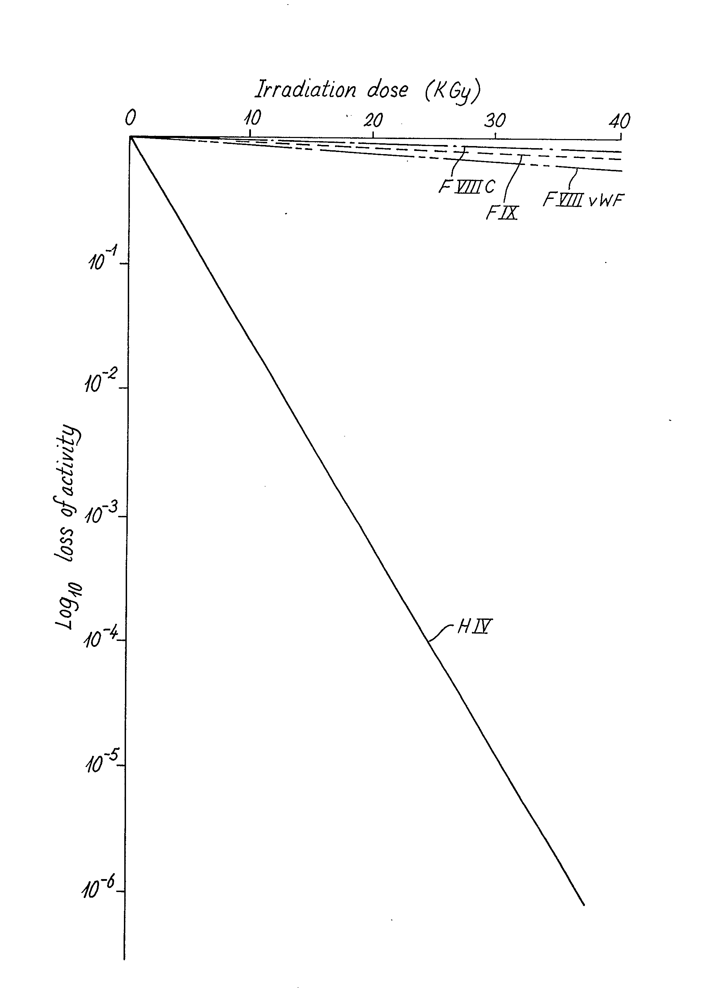

Following irradiation inoculated samples from the $U^{235}$ spent reactor fuel rods experiment were assayed for HIV infectivity using an assay procedure based on that of Weiss et al, Nature, 1986, 324, 572-574 which involved incubating equal replicate volumes of tenfold virus dilutions in RPM1 1640 medium containing 10% v/v foetal calf serum and C8166 indicator cells for 3-4 days. Two volumes of fresh medium were then added and after 7 days the cultures were examined for syncytia formation. The effects of the gamma irradiation on viral activity are shown in Table 1 where the $\log_{10}$ of the loss of activity is shown for each irradiation dose used, the linear regression data being quoted under the Table. The figure for the loss at a dose of 40 KGy is shown as greater than 4.3 since the sensitivity of the assay does not allow the detection of complete inactivation, a loss of 4.3 being the maximum level of inactivation which can be detected.

Uninoculated samples from the $U^{235}$ experiment and samples from the two cobalt 60 source experiments were assayed for coagulation factor activity, Factor VIII:C being assayed chromogenically using the Kabi Vitrum procedure with the manufacturer's microplate modification, Factor VIII:vWF being assayed for ristocetin cofactor activity using ristocetin induced platelet aggregation and Factor IX being assayed by a two stage automated partial thromboplastin test (APTT) using the Organon Teknika Coag-a-Mate coagulation analyser. The effects of the gamma irradiation on each of the three factors is shown in Table 2. The results are summed over all three experiments (1 x $U^{235}$, 2 x $Co^{60}$) and due to the variation of the irradiation doses in each experiment, the data from separate experiments was subjected to linear regression analysis, values being obtained for each coagulation factor at each of the target doses. Table 2 gives the mean of the results from each experiment with the inactivation rate being expressed as the $\log_{10}$ of the loss of activity per KGy in International Units (iu)/ml, the linear regression data being quoted under the Table.

The inactivation rates of both the HIV and the coagulation factors were found to follow first order kinetics. The tables indicate the differential between HIV inactivation and coagulation factor inactivation, the inactivation rate for HIV being 0.164 $TCID_{50}$ doses/ml/KGy ($TCID_{50}$ is a quantitative estimation of that viral titre, based on the dilution, which gives 50% positive and 50% negative responses), whilst the inactivation rates for FVIII:C, FVIII:vWF and FIX were 0.00173, 0.00526 and 0.00286 $\log_{10}$ units/ml/ KGy, corresponding to percentage activity loss rates of about 0.4, 1.13 and 0.66% activity/KGy respectively. The use of low temperatures for the irradiation is therefore seen to allow sufficient dosage levels of irradiation to inactivate the HIV without any unacceptable loss of activity in the coagulation factors. The differential between HIV inactivation and loss of clotting factor activity at the low temperature used in these experiments is clearly shown by the Figure in which $\log_{10}$ loss of activity is plotted against irradiation dose for HIV and all three factors.

Table 1

Effect of gamma irradiation on HIV activity

| Dose KGy | Loss in $\log_{10}$ Infectious doses/ml of HIV activity ($TCID_{50}$) |
|---|---|
| 0 | 0 |
| 2.5 | 0.2 |
| 5.0 | 1.0 |
| 10 | 1.8 |
| 20 | 3.2 |
| 40 | >4.3 |

Linear regression : n = 5, r = 0.992, p = > 0.01 and k = -0.164

Table 2

Effect of gamma irradiation on clotting factors

| Factor | Dose kGy | Loss of coagulant activity ($\log_{10}$ units) per KGy (iu/ml) | Percentage loss |
|---|---|---|---|
| Factor VIIIC | 0 | 0 | 0 |
| | 2.5 | 0.0046 | 1.1 |
| | 5 | 0.0083 | 1.9 |
| | 10 | 0.0202 | 4.5 |
| | 20 | 0.0367 | 8.1 |
| | 40 | 0.0688 | 14.6 |
| Factor VIII:vWF | 0 | 0 | 0 |
| | 2.5 | 0.0191 | 4.3 |
| | 5 | 0.0325 | 7.2 |
| | 10 | 0.0565 | 12.2 |
| | 20 | 0.1120 | 22.7 |
| | 40 | 0.2130 | 38.8 |
| Factor IX | 0 | 0 | 0 |
| | 2.5 | 0.00745 | 1.7 |
| | 5 | 0.0164 | 3.7 |
| | 10 | 0.0195 | 4.4 |
| | 20 | 0.0619 | 13.3 |
| | 40 | 0.115 | 23.3 |

Linear regression : n = 6 (all factors), r = 0.998 (VIIIC and IX) and 0.999 (VIII:vWF), p = > 0.05 (all factors) and k = -0.00173 (VIIIC), -0.00526 (VIII;vWF) and -0.00286 (IX).

**Claims**

1. A method for the treatment of blood plasma or a plasma derived product or other body tissues which comprises subjecting the plasma, tissue or derived product to gamma radiation at a temperature which is less than ambient temperature.

2. A method according to Claim 1 characterised in that the irradiation is effected at a temperature of 0°C or less.

3. A method according to either of the preceding Claims characterised in that the irradiation is effected at a temperature which is in the range -20°C to -60°C.

4. A method according to any of the preceding Claims characterised in that the radiation dose employed is in the range 20 to 60KGy.

5. A method according to any of the preceding Claims characterised in that the radiation dose employed is at least 40KGy.

6. A method according to any of the preceding Claims characterised in that the treatment is carried out on blood plasma or a plasma derived product.

7. A method according to Claim 6 characterised in that the treatment is carried out on a material which is a liquid at ambient temperature.

8. A method according to any of Claims 1 to 5 characterised in that the treatment is carried out on a human body tissue which is suitable for transplantation.

9. A method according to Claim 8 characterised in that the body tissue comprises non-viable cellular tissue.

10. A method according to either of Claims 8 or 9 characterised in that the body tissue is selected from the group comprising teeth and bone.

Irradiation dose (KGy)